(19) Europäisches Patentamt

European Patent Office

Office européen des brevets

(11) **EP 0 896 579 B1**

(12) **EUROPEAN PATENT SPECIFICATION**

(45) Date of publication and mention
of the grant of the patent:
**10.07.2002 Bulletin 2002/28**

(51) Int Cl.[7]: **C07K 1/32**, C12N 9/98,
C12N 9/24

(21) Application number: **97953171.2**

(22) Date of filing: **18.12.1997**

(86) International application number:
**PCT/US97/22796**

(87) International publication number:
**WO 98/30580 (16.07.1998 Gazette 1998/28)**

(54) **RECOVERY OF PROTEINS BY PRECIPITATION USING LIGNOSULFONATES**

RÜCKGEWINNUNG VON PROTEINEN DURCH FÄLLUNG MIT LIGNINSULFONATE

RECUPERATION DE PROTEINES PAR PRECIPITATION AU MOYEN DE LIGNOSULFONATES

(84) Designated Contracting States:
**AT BE CH DE DK ES FI FR GB GR IE IT LI NL PT
SE**

(30) Priority: **10.01.1997 US 781333**

(43) Date of publication of application:
**17.02.1999 Bulletin 1999/07**

(73) Proprietor: **GENENCOR INTERNATIONAL, INC.
Palo Alto, California 94304 (US)**

(72) Inventors:
• **BECKER, Nathaniel, T.
Burlingame, CA 94010 (US)**
• **LEBO, Stuart, E., Jr.
Schofield, WI 54476 (US)**

(74) Representative:
**Wibbelmann, Jobst, Dr., Dipl.-Chem. et al
Wuesthoff & Wuesthoff,
Patent- und Rechtsanwälte,
Schweigerstrasse 2
81541 München (DE)**

(56) References cited:
**FR-A- 2 205 532        US-A- 3 390 999
US-A- 3 622 510**

• **CHEMICAL ABSTRACTS, vol. 91, no. 11, 10
September 1979 Columbus, Ohio, US; abstract
no. 85240e, TADROS T F : "Adsorption of
lignosulfonates on pesticides for aqueous
solution and the stability of the resulting
dispersions" page 248; column 2; XP002063586
& ADV. PESTIC. SCI. PLENARY LECT. SYMP.
PAP. INT. CONGR. PESTIC CHEM. 4TH, vol. 3,
1978, OXFORD GB, pages 820-826,**
• **CHEMICAL ABSTRACTS, vol. 100, no. 2, 9
January 1984 Columbus, Ohio, US; abstract no.
8813w, POLISHCHUK S A ET AL: "Effect of
colloidal-chemical properties of anionic
surfactants on the quality of commercial forms
of organic dyes. III. Effect of the composition and
degree of sulfonation of sodium lignosulfonate
on its colliodal- chemical properties" page 8819;
column 1; XP002063587 & ZH. PRIKL. KHIM., vol.
56, no. 10, 1983, PETERSBURG SU, pages
2285-2289,**

## Description

### Field of the Invention

The present invention relates to recovering proteins from an aqueous solution using lignosulfonates that have a low degree of sulfonation.

### Background of the Invention

Sulfonated lignins are natural, phenolic polymers derived from the pulping of wood. They have long been used to recover proteins from process waste streams (1-4). They have also been used to isolate and stabilize peptide-based antibiotics (5).

The mechanism involved in all of these processes is not fully understood Kawamoto and coworkers (6) studied the protein absorbing capacities of various lignins and found no correlation between precipitation efficiency and lignin molecular weight. They also found no correlation between precipitation efficiency and the phenolic hydroxyl content.

U.S. Patent 3,035,919 discloses using alkali-soluble lignin protein to precipitate bacitracin from solution.

U.S. Patent 3,047,471 discloses a method of refining amyloglucosidase consisting of mixing an aqueous solution or dispersion of an amyloglucosidase preparation with a small proportion of a selected protein precipitant or coagulant. The mixture is then filtered, centrifuged or decanted to separate the liquid and solid portions. The liquid portion contains the treated or purified amyloglucosidase preparation. The protein precipitant can be lignin or tannic acid.

British Patent 1,092,628 discloses a process for the preparation of a proteinous animal food from proteinous waste water The waste water is treated with substantially pure high molecular weight lignosulfonic acids or torula yeast-fermented sulphite waste liquor. There is no indication as to what degree of sulfonation is present in the high molecular weight lignosulfonic acids.

U.S. Patent 3,616,235 discloses a process for producing a dry enzyme or protein preparation having a low salt content. The process is characterized by the feature that precipitation from an albumen-containing culture solution or culture filtrate is carried out with an anionic, cationic or amphoteric synthetic organic tanning agent and that subsequent extraction of inorganic salts and excess tanning agent takes place using water or mixtures of water and an organic solvent.

British Patent 1,202,254 discloses a process for removing proteins and any degradation products from waste water. The process includes precipitating the proteins and any degradation products, under acidic conditions, as a complex with an aryl or arylalkyl sulfonic acid or sulfonate and then separating the precipitated product.

U.S. Patent 3,622,510 discloses a process for the recovery of proteinaceous material from an aqueous plant effluent. The process includes treating the effluent with a low molecular weight lignosulfonate fraction at a pH below the isoelectric point of the proteinaceous materials to obtain a lignosulfonate-protein floc.

None of the literature discloses whether a certain degree of sulfonation is preferred. In fact, in many of the above patents, the lignosulfonic acid or lignosulfonate was added in the form of pulping liquor that had been treated to remove other constituents such as sugars. The resulting liquor contained lignosulfonate or lignosulfonic acids with varying degrees of sulfonation, and typically pulping liquor is highly sulfonated.

### Summary of the Invention

Current methods for isolation of enzymes and other proteins are equipment, time and capital intensive. There exists a need for an inexpensive, high yield process for concentrating, purifying and stabilizing proteins such as enzymes directly from fermentation broth, for example, cell-free fermentation broth. It has been found that lignosulfonates having a low degree of sulfonation are the preferred lignosulfonates for precipitating proteins, particularly enzymes.

The present invention provides a process for recovering protein from an aqueous solution. The process includes a) adding to the aqueous solution one or more lignosulfonates at a mass ratio of at least 0.1:1 (lignosulfonate to protein), the lignosulfonate having a degree of sulfonation of less than about 0.5; b) adjusting the pH of the solution of step a) to a pH of about 1-5 pH units less than the isoelectric point of the protein to be recovered to form an insoluble complex between the lignosulfonate and the protein; and optionally c) separating the complex of step b) from the aqueous solution.

Also provided are lignosulfonate/protein complexes such that the protein can be formulated either as a complex or after decomplexing from the lignosulfonate.

The protein is preferably a fungal or bacterial enzyme.

Further provided are methods of using the lignosulfonate/protein complexes to form a granule.

## Brief Description of the Drawings

Figure 1 shows the effect of lignosulfonate molecular weight on precipitation yield for a 2:1 lignin/protease mass ratio.

Figure 2 shows the effect of lignosulfonate degree of sulfonation on precipitation yield for a 2:1 lignin/protease mass ratio.

Figure 3 shows the yield of amylase complexed versus pH for two different lignosulfonates.

## Detailed Description of the Invention

In the primary embodiment of the invention, an aqueous solution of protein is contacted with an aqueous solution of lignosulfonate and the combined solutions are mixed. Preferably, the relative amount and pH of each solution are estimated beforehand to ensure that the final pH of the mixture is at or close to the desired pH for complexation. When this is done, yields appear to be better than when the pH of the mixture of lignosulfonate and protein is directly adjusted to the desired pH. (See Example 1.)

After the solutions have been mixed and the lignosulfonate has complexed with the protein, the complex is separated from the aqueous solution by, for example, centrifuging the mixture and collecting the pellet. The resulting complex can be formulated as is or can be further treated to decomplex the protein from the lignosulfonate

The types of aqueous solutions that can be used in the present invention include solutions produced during the production of a protein, for example, whole fermentation broth, cell free broth, centrate, ultrafiltered concentrate and column chromatography eluate.

In a preferred embodiment the protein to be complexed is an enzyme. The term "enzyme" includes proteins that are capable of catalyzing chemical changes in other substances without being changed themselves. Enzymes within the scope of the present invention include pullulanases, proteases, cellulases, amylases, isomerases, e.g., glucose isomerase, lipases, oxidases and reductases. In the present invention, the protein complexes with a lignosulfonate having a low degree of sulfonation. Using a lignosulfonate having a low degree of sulfonation, at least 80% of the protein is complexed from the solution Preferably at least 90% of the protein is complexed and most preferably, at least 95% of the protein is complexed.

Commonly, lignosulfonates or sulfonated lignins are obtained from the residual pulping liquors from the pulp and paper industry where lignocellulosic material such as wood, straw. or corn stalks is processed to separate the cellulose or pulp from the lignin. In the sulfite pulping process, the lignocellulosic material is digested with a sulfite or bisulfite to obtain a sulfonated residual pulping liquor commonly known as spent sulfite liquor" wherein the sulfonated lignin is dissolved In other processes, the residual pulping liquor as obtained from the pulping process may not be a sulfonated product However, the residual liquors or products containing the lignin portion of the lignocellulosic material from other processes and also from the sulfite process may be treated by various known methods to sulfonate the lignin to the different degrees desired

The degree of sulfonation is a measure of the sulfonate ($SO_3^=$) groups per phenylpropane monomer ($C_9$) unit of the lignin. A low degree of sulfonation provides very good protein recovery yields after complexing protein from a solution.

Phenylpropane is the basic monomer unit of the natural lignin polymer but it's exact derivitization varies from one natural source to another. Thus, the elemental composition varies among species of trees. For example, the $C_9$ formula for softwood/spruce is $C_9H_{8.83}O_{2.37}(OCH_3)_{0.96}$ and the $C_9$ formula for hardwood/birch is $C_9H_{9.03}O_{2.77}$ $(OCH_3)_{1.58}$. This gives molecular weights of 184.15 grams/mole softwood $C_9$ units and 210.33 grams/mole hardwood $C_9$ units.

Based on the literature, it is reasonable to assume is that all of the sulfonic sulfur in a given sample is associated with the lignosulfonate component. Using the lignosulfonate content of a given product as described by the manufacturer of that product, the sulfonation of the lignosulfonate can be determined. It should be noted that when $SO_3^=$ reacts with lignin, it links to a carbon atom in the polymer, typically producing either the sodium salt (molecular weight $SO_3Na$ = 103 grams/mole) or the calcium salt (molecular weight $SO_3Ca_{\frac{1}{2}}$ = 100 grams/mole) of the resulting lignosulfonate.

In the following calculations, the following abbreviations are used:

DS = degree of sulfonation
LSS = sulfonate sulfur content of lignosulfonate (g sulfonate sulfur/g lignosulfonate)
PS = sulfonate sulfur content of product (g sulfonate sulfur/g product)
PL = lignosulfonate content of the product (g lignosulfonate/g product)
LSSO = $SO_3Na$ content of lignosulfonate (g $SO_3Na$/g lignosulfonate)
LSMSO = moles of sulfonate salt (i.e., $SO_3Na$) per grams of lignosulfonate

LSL = equivalent lignin content of lignosulfonate (g lignin/g lignosulfonate)
GL = grams of lignin
ML = moles of lignin
M( ) = molecular weight of $C_9$ lignin unit

To calculate the degree of sulfonation (DS) of a given lignosulfonate, first the sulfonate sulfur content of the lignin component of the lignosulfonate is calculated by dividing the determined sulfonate sulfur content of the lignosulfonate by the lignin content of the lignosulfonate:

$$LSS = PS / PL$$

Next, the weight percentages of the sulfonate groups in the lignosulfonate are calculated and, for example, for a sodium-based product, the moles of $SO_3Na$/gram of lignosulfonate using the appropriate molecular weights (103 grams $SO_3Na$ and 32 grams/mole S):

$$LSSO = LSS * [M(SO_3Na) / M(S)] = LSS * (103/32)$$

$$LSMSO = LSSO / M(SO_3Na) = LSSO / 103$$

Now, assuming a basis of one gram of lignosulfonate, the calculation for the weight contribution from sulfonate groups is as follows:

$$LSL = 1 - LSSO$$

$$GL = (1\ gram\ lignosulfonate) * LSL$$

This must be done in order to use the originally assumed $C_9$ molecular weights. Finally, the number of moles of lignin are calculated by dividing the grams of lignin by the appropriate molecular weight and a DS unit value is obtained as follows:

$$DS = LSMSO / ML$$

For the purposes of the present invention, the degree of sulfonation of the lignosulfonate sample is less than about 0.5. Preferably, the degree of sulfonation is between 0.1 and 0.5.

For example for a softwood, sodium lignosulfonate sample having a sulfonate sulfur content of 2.35% and a lignosulfonate content of 90.2%, the calculation of the sample's degree of sulfonation is as follows.
    $SO_3Na$ content of lignosulfonate by weight and moles $SO_3Na$/gram of lignosulfonate :

(2.35% sulfonate sulfur)(90.2% lignosulfonate sulfur) = 2.60% sulfonate sulfur associated with lignosulfonate;
(2.60% sulfonate sulfur)(103 grams $SO_3Na$/32 grams S) = 8.39% $SO_3Na$ by weight;
(8.39% $SO_3Na$ by weight)(103 grams $SO_3Na$/mole) = 0.000817 moles $SO_3Na$/gram of lignosulfonate

The moles of lignin:

(1-0.0817% $SO_3Na$ by weight) *100 = 91.8% lignin by weight
(1 gram LS)(91.6% lignin by weight) = 0.916 grams of lignin,
0.916 grams of lignin/(185.15 grams/mole $C_9$ units) = 0.00487 mole $C_9$ units

$$DS = 0.00817/0.00487 = 0.168.$$

For the purposes of the present invention, the degree of sulfonation of the lignosulfonate sample is less than about 0 5 Preferably, the degree of sulfonation is between 0 1 and 0.5

Lignosulfonates of low sulfonation complex proteins even at the high ionic strengths (i.e., 5-20 mS/cm (5-20 mmho) typical of industrial fermentations Without wanting to be bound by theory, it would appear that the reduced degree of sulfonation allows hydrophobic interactions between the lignin and the protein to complement the ionic interactions which would otherwise be shielded at ionic strengths above about 2 mS/cm 2 (mmho).

Although the present invention works at high ionic strengths, in some instances it may be desirable to reduce the ionic strength of the solution by, e.g., dilution or diafiltration.

In the method of the invention, the pH of the lignosulfonate/protein solution is lowered at least one unit below the protein's isoelectric point. When the pH is lowered, the protein and lignosulfonate bind together to form a complex. As noted above, the lignosulfonate/protein complex probably forms as a result of both ionic interactions and hydrophobic interactions between the lignosulfonate and the protein.

The isoelectric point (pl) of a protein is the pH at which the protein carries no net electric charge. At the isoelectric point, the electrophoretic mobility is zero, i.e., the protein will not migrate in an electric field. Below the pl, the protein is positively charged; above the pl, the protein is negatively charged.

Electrophoretic mobility and pl can be determined for proteins in their native form by the technique of isoelectric focusing. The protein sample is run on an isoelectric focusing (I.E.F.) gel, using amphyolyte buffers to establish a pH gradient. Standardized pre-set I.E.F. gels can be purchased. Proteins migrate to points of high resolution on the gel, allowing the pH to be read off using standards or a calibrated gel. The technique is described in Robert Scopes, Protein Purification: Principles and Practice, Springer-Verlag, New York, 1982, pp. 172-177 and pp. 250-251.

The magnitude of the required difference between pH and pl will depend inversely on the degree of charge titrated on the protein for a given drop in pH.

The lignosulfonate/protein complex of the present invention exhibits improved storage stability. The complex is not merely a precipitated protein but represents a unique form of matter. The complex has excellent long-term storage ability, particularly when the moisture level is reduced to about 5% w/w or less, even at elevated temperatures. Drying of the complex can be carried out by, e.g., freeze-drying or spray-drying.

The lignosulfonate/protein complex can be decomplexed in a number of ways. For example, the complex can be suspended in water or an aqueous buffer and base can be added to raise the pH so as to facilitate decomplexation. The complex can also be suspended in an aqueous buffer and the complex diluted to such an extent as to facilitate decomplexation.

For example, a complex of lignosulfonate and enzyme can be broken most easily by suspending the complex in an aqueous solution and raising the pH above the isoelectric point. The exact degree of enzyme activity released into solution is a function of a unique titration curve for each protein and enzyme but, in general, complete dissociation can be expected 1-5 pH units above the pl of the protein.

An alternative means of decomplexation is to raise the ionic strength of a suspension of the complex. The low sulfonated lignosulfonates (such as D460-7) have less sensitivity to ionic strength than do the high sulfonated lignosulfonates (such as CBOS-6), and so generally will require very high levels of salt, greater than 80 mS/cm (80 mmho) in conductivity, to release more than 75% of the protein into solution.

Protein which has been released from a complex with lignosulfonate is generally still a cosolute since lignosulfonates are highly soluble and, in fact, act as a good solubilizer of proteins at a pH above the pl of the protein. It may be desirable to separate the lignosulfonate from the soluble protein if, for example, the dark color or other properties of the lignosulfonate would not be desired in a formulation. This can be done, in principle, by flocculating the lignosulfonate with a multivalent cation such as calcium, a cationic polyelectrolyte polymer such as a polyamine, or an anion exchange resin.

The protein purified using the present invention can be added to a formulation either as part of the complex or after decomplexation and removal of the lignosulfonates. For example, the lignosulfonate/protein complex can be added to detergent compositions either directly or as part of a granule.

The complex of the invention can be easily spray-coated onto inert carrier particles such as non-pareil seeds, salt grains or prills to form granules. For example, in a detergent formulation the pH stability of granules made using the complex are excellent since the complex exists stably at the granular formulation pH (around 5-6) but it is rapidly dissociated once the pH is raised to 9 or higher by contact with the alkaline detergent solution in the wash application.

The complex of the invention wherein the degree of sulfonation is between 0.1 and 0.5 can also be mixed with binders and granulated by extrusion, spheronization, or high intensity granulation.

## Experimental

Protease concentration was measured by a colorimetric spectrophotometer assay to measured/hydrolysis rate of a synthetic substrate. N-succinyl Ala-Ala-Pro-Phe p-nitroanilide according to Bonneau et al. (1991) *J. Am. Chem. Soc.* **113**(3):1030.

Initial Screening Procedure

A 6 g/L solution of lignosulfonate was adjusted to pH 3.0 with 10% formic acid. A 1:1 dilution of this solution was used to make a 3 g/L solution of lignosulfonate at pH 3.0. 3.0 mL of the lignosulfonate solution were placed into a 15 mL centrifuge tube. 3.0 mL of protease, pH 5.0, were added to the tube and mixed for 10 seconds on a vortex mixer. The material was centrifuged for 5 minutes at 2200 rpm and the pH and the conductivity of the supematant was measured. A 1:1000 dilution of the supematapt was made and the Protease Assay was performed as described in the assay procedure.

Sodium Chloride Level Experiment

1. Preparation of Protease

The appropriate amount of sodium chloride was added to 50 mL of raw protease and the pH was adjusted to 5.0 with 10% formic acid. Solutions of 0%, 1%, 2%, 5% and 10% sodium chloride were made. It was assumed that raw protease does not contain significant amounts of sodium chloride.

2. Sample Preparation

    (a) 3.0 mL of lignin solution at 6 g/L and pH 3.0 were placed into five 15 mL centrifuge tubes.
    (b) 3.0 mL of protease with 0% sodium chloride were added to one of the centrifuge tubes and mixed for 10 seconds.
    (c) The tube was centrifuged for 5 minutes at 2200 rpm.
    (d) The pH of the supernatant in the tube was measured and discarded. The pellet was then resuspended in enzyme diluent.
    (e) A 1:1000 dilution of the resuspended pellet solution was made.
    (f) Steps 2 - 5 were repeated for protease with 1%, 2%, 5% and 10% sodium chloride.
    (g) The protease assay was performed on all of the samples as described in the assay procedure.

Example 1

**Examples of Lignosulfonate Molecular Weight and Degree of Sulfonation**

Using subtilisin protease produced according to the disclosure of WO 95/10615, the precipitation efficiencies of 23 commercial and experimental lignosulfonates were determined at lignosulfonate:protease enzyme mass ratios of 1:1 and 2:1. The samples were prepared as outlined above. The lignosulfonates tested had a wide range of molecular weights and varying degrees of sulfonation (see Table 1). The supernatant based precipitation efficiencies of these products ranged from 0-66 % at a 1:1 lignin:enzyme ratio to 9-93 % at a 2:1 lignin:enzyme mass ratio (see Table 1). While conductivity did not seem to affect precipitation efficiency, pH did. While the reason for it is not clear, it was also observed that addition of lignin preadjusted to pH 3 to protease preadjusted to pH 5 was more efficient than directly adjusting the pH of a mixture of lignin and protease to the same final pH (see Table 2).

Table 1

| Lignin Precipitation Database - Protease | | | | | | | | | |
|---|---|---|---|---|---|---|---|---|---|
| Lignin | Lignin Physical Properties | | | Precipitation Results | | | | | |
| | pH | $SO_3/C_9$ | Mn | 2:1 | | | 1:1 | | |
| | | | | Yield | pH | Cond. | Yield | pH | Cond. |
| Marasperse™ CBOS-6 | 10.0 | 1.10 | 35000 | 9 | 4.0 | 6.18 | 41 | 4.2 | 5.78 |
| Marasperse™ B-3D | 5.7 | 0.8 | 47500 | 17 | 4.3 | 5.53 | 14 | 4.6 | 5.33 |
| Marasperse™ CBA-1 | 9.6 | 0.23 | 35600 | 90 | 3.9 | 5.97 | 46 | 4.1 | 5.64 |
| Maracarb™ N-1 | 8.4 | 1.48 | 5500 | 10 | 3.9 | 6.17 | 0 | 4.1 | 5.74 |
| Dynasperse™ LCD | 10.8 | 0.62 | 58000 | 80 | 3.8 | 6.19 | 30 | 4.0 | 5.73 |

Table 1   (continued)

| Lignin | Lignin Physical Properties | | | Precipitation Results | | | | | |
|---|---|---|---|---|---|---|---|---|---|
| | pH | SO$_3$/C$_9$ | Mn | 2:1 | | | 1:1 | | |
| | | | | Yield | pH | Cond. | Yield | pH | Cond. |
| Ufoxane™ 3A | 8.5 | 0.49 | 57800 | 71 | 4.1 | 5.69 | 40 | 4.3 | 5.69 |
| Marasperse™ 52CP | 9.9 | 0.87 | 51000 | 65 | 3.8 | 6.3 | 31 | 4.0 | 5.71 |
| D-451-9 | 8.5 | 0.17 | 36000 | 93 | 3.9 | 5.79 | 26 | 4.2 | 5.21 |
| D-453-7 | 11.0 | 0.65 | 60000 | 84 | 3.9 | 6.15 | 48 | 3.9 | 5.80 |
| D-453-8 | 10.8 | 0.65 | 40000 | 55 | 3.8 | 6.06 | 26 | 3.9 | 5.74 |
| D-453-9 | 10.0 | 1.20 | 45000 | 13 | 4.1 | 5.73 | 15 | 4.0 | 5.84 |
| D-453-10 | 10.0 | 0.45 | 44000 | 62 | 3.8 | 6.27 | 38 | 3.8 | 6.04 |
| D-460-7 | 9.6 | 0.30 | 36000 | 64 | 4.2 | 5.95 | 51 | 4.2 | 5.79 |
| D-468-5 | 4.3 | 0.65 | (120000) | 47 | 4.0 | 5.15 | 47 | 4.0 | 5.15 |
| D-468-6 | 7.0 | 0.55 | (120000) | 40 | 4.0 | 4.89 | 40 | 4.0 | 4.89 |
| D-468-7 | 4.3 | 0.65 | 45500 | 66 | 4.0 | 5.05 | 66 | 4.0 | 5.05 |
| D-468-9 | 10.3 | 0.24 | 60000 | 76 | 4.1 | 5.70 | 11 | 4.2 | 5.66 |
| D-468-10 | 10.5 | 0.29 | 61300 | 83 | 3.8 | 6.25 | 32 | 4.0 | 5.98 |
| D-468-11 | 10.5 | 0.36 | 43900 | 85 | 3.9 | 6.09 | 46 | 4.0 | 5.92 |
| N/A = not available: Numbers in parentheses are estimates | | | | | | | | | |

Table 2

| Effect of Order of Addition on Precipitation Yield (Lignin:Protease Ratio = 2:1) | | |
|---|---|---|
| Precipitation Yield | | |
| Lignin | pH 3 Lignin + pH 5 Protease | pH Adjusted Lignin/Enzyme Mix |
| D-451-9 | 93 | 89 |
| D-468-10 | 83 | 65 |
| D468-11 | 85 | 69 |

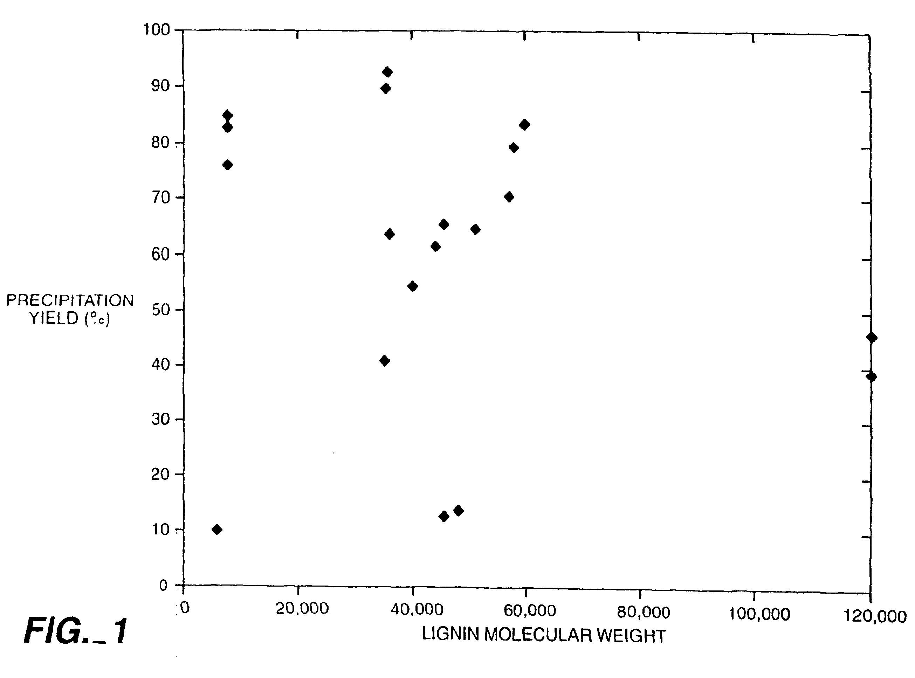

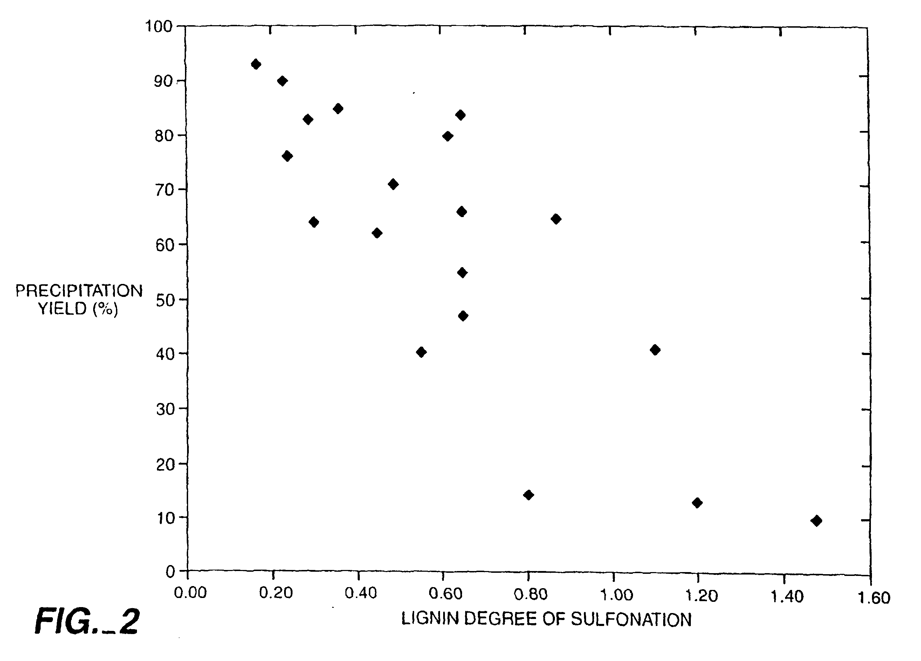

Further analysis of the 2:1, lignin:enzyme data indicated that there was little correlation between lignin molecular weight and precipitation efficiency (see Figure 1). A correlation was found between degree of sulfonation and precipitation efficiency (see Figure 2). Lignin products with low degrees of sulfonation gave precipitation efficiencies of 80% or better.

Effect of Sodium Chloride on Precipitation Efficiency

The effect of sodium chloride on precipitation efficiency was determined for three products ranging in degree of sulfonation from 0.17 to 0.65 SO$_3$/C$_9$ unit using the method described in the Materials and Methods section above. Specifically, D-451-9 (SO$_3$/C$_9$ unit = 0.17), D-468-11 (SO$_3$/C$_9$ unit = 0.36) and D-453-7 (SO$_3$/C$_9$ unit = 0.65) were tested.
For all three products, a general trend towards lower yield with increasing sodium chloride concentration was observed. At sodium chloride concentrations greater than 20 g/l, precipitation efficiencies dropped to less than 65% for all three products. The effect of sodium chloride also seemed to be independent of the degree of sulfonation of the lignin although it did appear that yields in general were slightly higher with D-451-9.

Example 2

**Reproducibility**

The reproducibility of yields determined via the supernatant procedure described in the Initial Screening Procedure section was determined by running five replicates of three different lignin products.

Table 3

| Lignin | Yield | | | | | Average | Standard Deviation |
|---|---|---|---|---|---|---|---|
| | #1 | #2 | #3 | #4 | #5 | | |
| D-451-9 | 90 | 94 | 94 | 94 | 94 | 93.2 | 1.8 |
| D-453-7 | 81 | 87 | 89 | 87 | 88 | 86.4 | 3.1 |
| D-468-11 | 82 | 85 | 88 | 89 | 89 | 86.6 | 3.1 |

Reproducibility Results

Example 3

**Mass Balance Data**

The use of the supernatant method for yield determination was verified by measuring the activities of the supernatant and of the redissolved pellet for four lignosulfonate/protease samples. In general, a good correlation was found between the yield obtained by supernatant measurements and that obtained by measurements of redissolved pellets (see Table 4)

Table 4

| Lignin | % Activity in Supernatant | % Activity in Pellet | Total Activity |
|---|---|---|---|
| D-451-9 | 2 | 97 | 99 |
| D-468-11 | 13 | 88 | 101 |
| D-468-13 | 17 | 88 | 105 |
| Maras. B-3D | 77 | 29 | 106 |

Mass Balance Data

Example 4

**Effect of pH on Complexation**

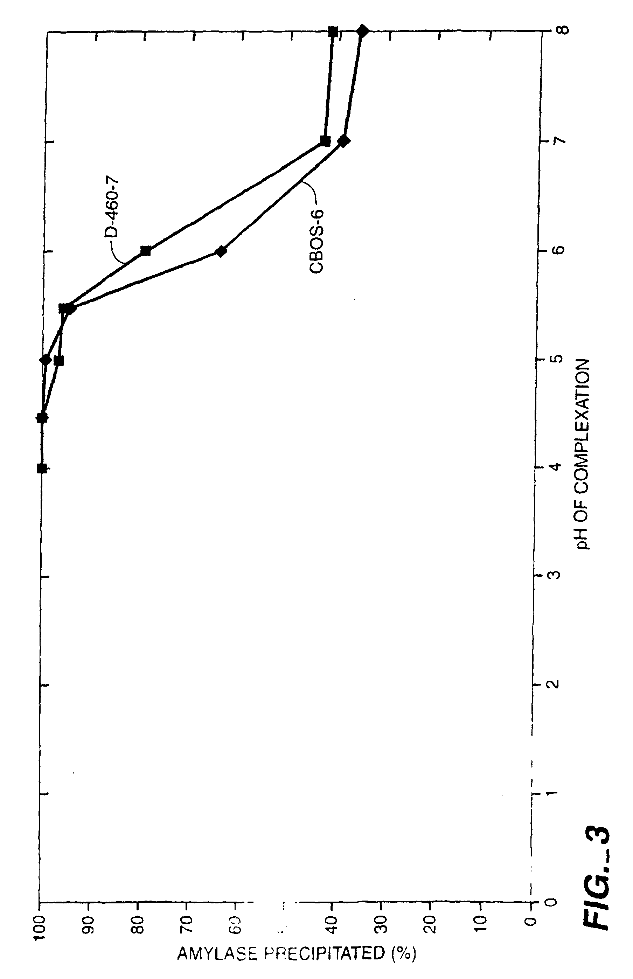

An amylase that was made according to the disclosure of WO 95/05295 complexed with two lignosulfonates, C-BOS and D-460-7, and the effect of pH on complexation was examined. 9.18 g/l of the amylase centrate were mixed with each lignosulfonate at a 2:1 lignosulfonate:enzyme mass ratio. The pH was adjusted to 8 with sodium hydroxide and then adjusted downward through additions of 10% formic acid. At each pH, the slurries were centrifuged and the centrates were assayed for amylase activity using the soluble substrate assay described in WO 94/05295. Figure 3 shows the percentage of amylase complexed as amylase lignosulfonate as a function of pH.

Example 5

**Granulation**

Lignosulfonate protease complex was prepared as a paste. The protease used was the same as that used in Example 1. 350 liters of 2.6 g/L filtered protease centrate were stirred with an overhead mixer. To this solution was added 9.1 kg of a 20% w/w solution of Lignosulfonate D-1030, resulting in a 2:1 weight ratio of lignin to active enzyme. [Calculation: 350L x 0.26% protein / 100 * 2:1 lignosulfonate:enzyme /0.20 = 9.1 kg.] The solution was pH adjusted to 4.8 with 0.5 liters 88% formic acid, causing the lignosulfonate enzyme complex to form as a slurry. 60 liters of this slurry were centrifuged for seven minutes at 3000 rpm in a Sharples™ 3PP350 solid bowl centrifuge. The centrate was assayed at 0.44 g/L protein. The sludge was removed and assayed at 90 g/kg protease, with a dry weight of 25.1% w/w solids. So the protease enzyme represents 9.0/25.1 = 36% of the total solids in the lignin-enzyme paste.

This 60 liters of the slurry were processed, giving 1.087 kg paste. The yield was: (90*1.087)/(2.6*60) = 63% yield. The low yield was partially due to handling losses.

To 784 grams of the above 90 g/kg paste were added 508 grams of a 59.5% w/w solution of Norlig™ A lignosulfonate. The slurry was allowed to mix several minutes.

751 grams of granular sodium sulfate cores were charged into a Vector Flo-Coater™ and fluidized at 60°C bed temperature. The above 1292 g protein slurry (containing 70.6 g active protein enzyme) was sprayed onto the cores at an initial rate of 20 g/minute, ramping up to 38 g/minute, with an inlet temperature of 76-81°, an outlet temperature of 44-52°, an atomization air pressure of 4-5 bar and an airflow of 3.9-4.5 $m^3$/sec (8300-9500 cfm). After enzyme application, a standard Enzoguard™ protective granule coating was applied as described in U.S. Patent 5,324,659. Enzoguard™ is an attrition-resistant polymer coating, including polyvinyl alcohol, nonionic surfactant and titanium dioxide pigment.

From the coater, 1.491 kg of granules with an activity of 41.7 g/kg protein were harvested, representing an 88.2% yield of active enzyme.

Example 6

**Detergent Formulation/Stability**

The storage stability of the granule made in Example 6 (Sample 1) and a granule made in a similar manner using a protease that can be made according to the disclosure of WO 91/06637 (Sample 3) were compared to control granules made using the same proteases that had not been complexed with lignosulfonate (Samples 2 and 4, respectively).

Each of the granules were formulated into a powder detergent with bleach at levels sufficient to deliver 0.11 ppm active enzyme to the wash. The fully formulated powder was stored in open containers at 26.7°C (80°F)/60% relative humidity for up to 6 weeks. At regular intervals, samples of the product were removed from storage and tested for cleaning performance and retained enzyme activity.

The wash test results showed that after six weeks of storage, all the samples were essentially identical in both cleaning performance and retained enzyme activity performance.

Example 7

**Xylanase**

Xylanase is an enzyme used by the poultry industry to improve the efficiency of the feed. The enzyme is normally prepared as a premix and blended in with the feed. The finished feed is pelletized for consumption by the chickens. The pelleting process involves injection of steam into the dyes where the feed is extruded. This process reduces the bioburden and releases the starches which act as binder.

Three batches of premix containing xylanase enzyme (GC140, commercially available from Genencor International, Inc.) were prepared using fluid bed granulation process. Two batches out of the three contained xylanase enzyme complexed with a lignosulfonate, one premix batch with non-complexed xylanase would be used as a control. Formulation:

| | Lot 1 | |
|---|---|---|
| Xylanase | | 367.6 ml |
| Lignosulfonate (D-460-7) | | 147 ml |
| Wheat Carrier | | 596 g |
| | Lot 2 | |
| Xylanase | | 367.6 ml |
| Wheat Carrier | | 632 ml |
| Xylanase | Lot 3 | 367.6 ml |
| Lignosulfonate (D-460-7) | | 73.52 ml |
| Wheat Carrier | | 596 g |

Procedure:

Manufacturing of premix with the complexed xylanase involved a two step process.

Step #1. Complexation: This step involved slow addition of lignosulfonate to the liquid xylanase while stirring. A pH adjustment was required during this process to provide a pH range where the xylanase enzyme is stable. This was achieved by addition of formic acid during the lignosulfonate/xylanase complex formation.

Step #2. Premix: One batch of the premix was prepared using the uncomplexed xylanase and the other two contained lignosulfonate/xylanase complex Lot 1 was prepared by blending the complexed xylanase with the wheat carrier in a Hobart mixer and drying the blend in a fluid bed dryer. The other two lots were manufactured by spraying the lignosulfonate/xylanase on to the carrier in a fluid bed granulator.

**References**

1. J. Wallerstein and E. Farber (1944) *Indust. and Eng. Chem.* **36**(8):772-774
2. U.S. Patent 2,418,311 (1947) W. McFarlane and N. Nikolaiczuk
3. U.S. Patent 3.390,999 (1964) L. Jantzen
4. A. Hopwood and G. Rosen (March 1972) *Process Biochem.* 15-17
5. U.S. Patent 3,053,919 (1962) J. Ziffler and T Cairney
6. H. Kawamoto, F. Nakatsubo and K. Murakami (1992) *Mokuzai Gakkaishi* **38**(1) 81-84

**Claims**

1. A process for recovering a protein from an aqueous solution, the process comprising:

   (a) adding to the aqueous solution one or more lignosulfonate(s) at mass ratio of at least 0.1:1 (lignosulfonate to protein ), the lignosulfonate having a degree of sulfonation less than about 0.5;
   (b) adjusting the pH of the solution of step (a) to a pH of about 1-5 pH units less than the isoelectric point of the protein to be recovered to form an insoluble complex between the lignosulfonate and the protein ; and
   (c) separating the insoluble complex of step (b) from the aqueous solution.

2. A process of Claim 1, wherein the degree of sulfonation is between 0.1 and 0.5.

3. A process of Claim 1, wherein the pH is adjusted to a pH of about 2-4 pH units less than the isoelectric point of the protein.

4. A process of Claim 1, wherein the protein is a fungal or bacterial enzyme.

5. A process of Claim 4, wherein the enzyme is selected from the group consisting of amylase, protease, cellulase, lipase, xylanase, pullulanase. reductase, oxidase, and glucose isomerase.

6. A process of Claim 1 further comprising the step of separating the lignosulfonate and the protein components of the complex.

7. A lignosulfonate/protein complex wherein the lignosulfonate has a degree of sulfonation less than about 0.5

8. A lignosulfonate/protein complex of Claim 7, wherein the degree of sulfonation is between 0.1 and 0.5.

9. A lignosulfonate/protein complex of Claim 7, wherein the protein is a fungal or bacterial enzyme.

10. A lignosulfonate/protein complex of Claim 7, wherein the enzyme is selected from the group consisting of amylase, protease, cellulase, lipase, xylanase, pullulanase, reductase, oxidase, and glucose isomerase

11. A lignosulfonate/protein complex of Claim 7 which is directly formulated into a liquid granular, powdered or paste protein-containing formulation.

12. A lignosulfonate/protein complex of Claim 8, wherein the complex is formulated using an organic or inorganic salt, polyol, organic polymer or a surfactant

13. A lignosulfonate/protein complex of Claim 8, wherein the complex is coated onto a core or particle to form a granule containing said complex

14. A lignosulfonate/protein complex of Claim 8, wherein the complex is mixed with binders and granulated by extrusion, spheronization or high intensity granulation

15. A lignosulfonate/protein complex of Claim 8, wherein the complex is directly agglomerated into a surfactant paste or other dry detergent composition.

16. A method of using the complex of Claim 7 to form a protein-containing granule, the method comprising coating the complex onto the surface of a core or particle and optionally further coating said particle

**Patentansprüche**

1. Verfahren zur Gewinnung eines Proteins aus einer wässrigen Lösung, wobei das Verfahren umfasst:

(a) Zugabe von einem oder mehreren Lignosulfonat(en) bei einem Massenverhältnis von mindestens 0,1:1 (Lignosulfonat zu Protein) zu der wässrigen Lösung, wobei das Lignosulfonat einen Sulfonierungsgrad von weniger als etwa 0,5 aufweist;
(b) Einstellen des pH der Lösung von Schritt (a) auf einen pH von etwa 1 - 5 pH-Einheiten unter dem isoelektrischen Punkt des zu gewinnenden Proteins, um einen unlöslichen Komplex zwischen dem Lignosulfonat und dem Protein zu bilden; und
(c) Abtrennen des unlöslichen Komplexes von Schritt (b) aus der wässrigen Lösung.

2. Verfahren nach Anspruch 1, in dem der Sulfonierungsgrad zwischen 0,1 und 0,5 liegt.

3. Verfahren nach Anspruch 1, in dem der pH auf einen pH von etwa 2 - 4 pH-Einheiten unter dem isoelektrischen Punkt des Proteins eingestellt wird.

4. Verfahren nach Anspruch 1, in dem das Protein ein Pilz- oder Bakterienenzym ist.

5. Verfahren nach Anspruch 4, in dem das Enzym ausgewählt ist aus der Gruppe bestehend aus Amylase, Protease, Cellulase, Lipase, Xylanase, Pullulanase, Reductase, Oxidase und Glucoseisomerase.

6. Verfahren nach Anspruch 1, weiter umfassend den Schritt des Trennens der Lignosulfonat- und der Proteinkomponente des Komplexes.

7. Lignosulfonat/Protein-Komplex, in dem das Lignosulfonat einen Sulfonierungsgrad von weniger als etwa 0,5 aufweist.

8. Lignosulfonat/Protein-Komplex nach Anspruch 7, in dem der Sulfonierungsgrad zwischen 0,1 und 0,5 liegt.

9. Lignosulfonat/Protein-Komplex nach Anspruch 7, in dem das Protein ein Pilz- oder Bakterienenzym ist.

10. Lignosulfonat/Protein-Komplex nach Anspruch 7, in dem das Enzym ausgewählt ist aus der Gruppe bestehend aus Amylase, Protease, Cellulase, Lipase, Xylanase, Pullulanase, Reductase, Oxidase und Glucoseisomerase.

11. Lignosulfonat/Protein-Komplex nach Anspruch 7, der direkt in eine flüssige, granuläre, pulverförmige oder pastenförmige Protein-haltige Formulierung formuliert ist.

12. Lignosulfonat/Protein-Komplex nach Anspruch 8, in dem der Komplex unter Verwendung eines organischen oder anorganischen Salzes, Polyols, organischen Polymers oder eines Tensids formuliert ist.

13. Lignosulfonat/Protein-Komplex nach Anspruch 8, in dem der Komplex einen Kern oder ein Teilchen überzieht, um eine Granalie zu bilden, die den Komplex enthält.

14. Lignosulfonat/Protein-Komplex nach Anspruch 8, in dem der Komplex mit Bindemitteln gemischt und durch Extrusion, Spheronisierung oder Hochintensitäts-Granulation granuliert ist.

15. Lignosulfonat/Protein-Komplex nach Anspruch 8, in dem der Komplex direkt in eine Tensid-Paste oder andere trockene Detergens-Zusammensetzung agglomeriert ist.

16. Verfahren zur Verwendung des Komplexes nach Anspruch 7, um eine Protein-haltige Granalie zu bilden, wobei das Verfahren das Auftragen des Komplexes auf die Oberfläche eines Kerns oder Teilchens und gegebenenfalls ein weiteres Beschichten des Teilchens umfasst.

## Revendications

1. Procédé de récupération d'une protéine dans une solution aqueuse, le procédé comprenant :

   (a) l'ajout à la solution aqueuse d'un ou de plusieurs lignosulfonate(s) à un rapport de masse d'au moins 0,1: 1 (lignosulfonate à protéine), le lignosulfonate ayant un degré de sulfonation inférieur à environ 0,5 ;
   (b) l'ajustement du pH de la solution de l'étape

   (a) à un pH d'environ 1 à 5 unités de pH inférieur au point isoélectrique de la protéine à récupérer pour former un complexe insoluble entre le lignosulfonate et la protéine ; et
   (c) séparation du complexe insoluble de l'étape (b) de la solution aqueuse.

2. Procédé de la Revendication 1, dans lequel le degré de sulfonation est compris entre 0,1 et 0,5.

3. Procédé de la Revendication 1, dans lequel le pH est ajusté à un pH d'environ 2 à 4 unités de pH inférieur au point isoélectrique de la protéine.

4. Procédé de la Revendication 1, dans lequel la protéine est une enzyme bactérienne ou fongique.

5. Procédé de la Revendication 4, dans lequel l'enzyme est sélectionnée dans le groupe consistant en amylase, protéase, cellulase, lipase, xylanase, pullulanase, réductase, oxydase, et glucose isomérase.

6. Procédé de la Revendication 1, comprenant en outre l'étape de séparation des composants du lignosulfonate et de la protéine du complexe.

7. Complexe lignosulfonate/protéine dans lequel le lignosulfonate a un degré de sulfonation inférieur à environ 0,5.

8. Complexe lignosulfonate/protéine de la Revendication 7, dans lequel le degré de sulfonation est compris entre 0,1 et 0,5.

9. Complexe lignosulfonate/protéine de la Revendication 7, dans lequel la protéine est une enzyme bactérienne ou fongique.

10. Complexe lignosulfonate/protéine de la Revendication 7, dans lequel l'enzyme est sélectionnée dans le groupe consistant en amylase, protéase, cellulase, lipase, xylanase, pullulanase, réductase, oxydase, et glucose isomérase.

11. Complexe lignosulfonate/protéine de la Revendication 7 qui est directement formulé en une formulation contenant une protéine en pâte, pulvérisée, ou granulaire liquide.

12. Complexe lignosulfonate/protéine de la Revendication 8, dans lequel le complexe est formulé en utilisant un sel organique ou inorganique, un polyol, un polymère organique ou un agent tensio-actif.

13. Complexe lignosulfonate/protéine de la Revendication 8, dans lequel le complexe est déposé sur un noyau ou une particule pour former un granule contenant ledit complexe.

**14.** Complexe lignosulfonate/protéine de la Revendication 8, dans lequel le complexe est mélangé avec des liants et granulé par extrusion, sphéronisation ou granulation à forte densité.

**15.** Complexe lignosulfonate/protéine de la Revendication 8, dans lequel le complexe est directement aggloméré en une pâte tensio-active ou en une autre composition détergente sèche.

**16.** Procédé d'utilisation du complexe de la Revendication 7 pour former un granule contenant de la protéine, le procédé comprenant le dépôt du complexe sur la surface d'un noyau ou d'une particule et facultativement le revêtement ultérieur de ladite particule.

FIG._1

EP 0 896 579 B1

PRECIPITATION YIELD (%)

LIGNIN MOLECULAR WEIGHT

FIG._2

FIG._3

pH OF COMPLEXATION

AMYLASE PRECIPITATED (%)

D-460-7

CBOS-6